# EUROPÄISCHE PATENTSCHRIFT

(11) **EP 3 685 143 B1**
(45) Veröffentlichungstag und Bekanntmachung des Hinweises auf die Patenterteilung: **29.11.2023**
(21) Anmeldenummer: 18773759.8
(22) Anmeldetag: 21.09.2018
(51) Int. Cl.: G01N 15/14, G01N 21/05

(54) **VERFAHREN ZUM VERFORMEN VON DEFORMIERBAREN KÖRPERN UND VORRICHTUNGEN DAZU**
METHOD FOR DEFORMING DEFORMABLE BODIES, AND DEVICES FOR THIS PURPOSE
PROCÉDÉ DE MISE EN FORME DE CORPS DÉFORMABLES ET DISPOSITIFS ASSOCIÉS

(30) Priorität: 23.09.2017 DE 102017008946
(43) Veröffentlichungstag der Anmeldung: 29.07.2020
(73) Patentinhaber: Universität Greifswald, 17489 Greifswald (DE)
(72) Erfinder: OTTO, Oliver, 17489 Greifswald (DE); CZERWINSKI, Fabian, 14057 Berlin (DE); FREGIN, Bob, 17493 Greifswald (DE)
(74) Vertreter: Hoffmann Eitle
(86) Internationale Anmeldenummer: PCT/EP2018/075605
(87) Internationale Veröffentlichungsnummer: WO 2019/057894

(56) Entgegenhaltungen:
- WO-A1-2015/024690
- US-A1- 2016 202 172
- US-A1- 2017 003 273
- ANDREJ POHAR ET AL: "Parallel flow of immiscible liquids in a microreactor: modeling and experimental study", MICROFLUIDICS AND NANOFLUIDICS, Bd. 12, Nr. 1-4, 27. August 2012 (2012-08-27), Seiten 307-316, XP055528506, DE ISSN: 1613-4982, DOI: 10.1007/s10404-011-0873-7
- Ma Shaohua ET AL: "Deformation of double emulsions under conditions of flow cytometry hydrodynamic focusing", Lab on a Chip, vol. 15, no. 22, 1 January 2015 (2015-01-01), pages 4291-4301, XP055781167, UK ISSN: 1473-0197, DOI: 10.1039/C5LC00693G Retrieved from the Internet: URL:https://pubs.rsc.org/en/content/articl epdf/2015/lc/c5lc00693g>

## Beschreibung

### Technisches Gebiet

Die vorliegende Erfindung betrifft ein Verfahren zum Verformen von deformierbaren Körpern, insbesondere Tröpfchen oder Zellen. Sie betrifft ferner ein Verfahren zum Bestimmen der mechanischen Eigenschaften von deformierbaren Körpern, bevorzugt Zellen.

### Stand der Technik

Die Analyse von biologischen Zellen wird oft dadurch erreicht, dass verschiedene fluoreszierende Farbstoffe selektiv an die Zelle gebunden werden. Eine Möglichkeit besteht darin, Farbstoffe an Antikörper zu binden. Diese binden an Oberflächenproteine, wenn diese auf einer Zelle vorhanden sind. Die Zelle ist dann mit diesem Farbstoff markiert. Die jeweils gesuchten Zelltypen können so mithilfe eines geeigneten Mikroskops selbst in heterogenen Proben nachgewiesen werden.

Bei Zellsuspensionen ist hierbei der Einsatz des von Dittrich und Göhde 1968 entwickelten Durchflusszytometers üblich, welches in der DE 1 815 352 beschrieben wird. Hierbei werden die Zellen einzeln nacheinander auf das Vorhandensein von Farbstoffen untersucht. Gleichzeitig besteht auch die Möglichkeit, die Zellen dem Messergebnis nach zu sortieren (Orfao A. et al., Clin. Biochem. (1969), Tung Y. C. et al., Sens. Actuators, B (2004)). Als Markierung werden auch magnetische Nanopartikel mit entsprechenden Antikörpern verwendet, sodass Zellen durch Magnetfelder sortiert werden können (sogenanntes MACS).

In den letzten Jahrzehnten haben sich außerdem Technologien etabliert, die den Fokus der Analyse von einer molekularen hin zu einer mechanischen Charakterisierung der Zelle verschieben. Diese Ansätze beruhen darauf, Folgen zu klassifizieren, die aufgrund von Änderungen im dynamischen Polymernetzwerk der Zelle (Cytoskeleton) auftreten. Das Cytoskeleton durchspannt die gesamte Zelle und ist für viele wichtige Zellprozesse von besonderer Bedeutung. Im Gegensatz zu molekularen Methoden erlaubt eine zellmechanische Charakterisierung eine hypothesenfreie Analyse, d. h. eine Analyse aufgrund eines inhärenten Markers.

Im Moment sind verschiedene Technologien zur mechanischen Analyse verfügbar, nämlich die Rasterkraft-Mikroskopie, Laser-basierte Kraftspektroskopie und Mikropipetten. Diese Methoden bestimmen die mechanischen Eigenschaften von Zellen aufgrund einer Verformung unter vorgegebener Kraft. Es ist möglich, in etwa 100 Zellen pro Stunde zu analysieren, was dazu führt, dass entsprechende Verfahren für kleinere Zellpopulationen geeignet sind. Es ist jedoch nicht möglich, diese Verfahren aufgrund der geringen Durchsatzraten auf die Anwendungsforschung zu übertragen.

Eine Weiterentwicklung dieser Technologien besteht in der hydrodynamischen Verformung. 2015 wurde mit der Real-Time Deformability Cytometry (RT-DC) eine Methode veröffentlicht, die eine mechanische Charakterisierung von Zellen mit bis zu 1000 Zellen pro Sekunde in Echtzeit ermöglicht (0. Otto et al., Real-Time Deformability Cytometry: On the Fly Mechanical Phenotyping. Nature Methodes, 12, 199-202 (2015) und WO 2015/024690 A1).

Bei RT-DC werden Zellen mit einer Spritzenpumpe durch einen mikrofluidischen Kanal gepumpt. Dieser Kanal hat einen Durchmesser, der den jeweiligen Zelldurchmesser um wenige Prozent übersteigt. Aufgrund der hydrodynamischen Wechselwirkung verformen Scher- und Normalkräfte die Zellen im Kanal. RT-DC hat sich als vorteilhaft zur Beantwortung grundlegender Fragen der Lebenswissenschaften erwiesen und ermöglicht eine Vielzahl von Anwendungen, die sowohl mit klassischer Durchflusszytometrie als auch mit klassischen mechanischen Verfahren unmöglich sind. Für die Details dieser Technologie wird auf die WO 2015/024690 A1 verwiesen.

### Technische Aufgabe

Den Erfindern sind verschiedene Probleme im Stand der Technik aufgefallen.

Was die bereits erwähnten Verfahren angeht, die Antikörperbasiert sind, sind Schwachstellen, dass z. B. das zellspezifische Oberflächenprotein für die Markierung bekannt sein muss. Ferner ist es nötig, die Zellsuspension mit vergleichsweise teuren Fluoreszenzfarbstoffen vorzubehandeln. Außerdem verändern die Zellen durch Fremdstoffe (wie z.B. die Farbstoffe) ihre Eigenschaften und die Zellsuspensionen werden außerdem mit fremden Stoffen kontaminiert. Letzteres kann dazu führen, dass diese Suspensionen hinterher nicht mehr bei Patienten eingesetzt werden können und entsorgt werden müssen, da die Farbstoffe u.U. für Patienten schädlich sein könnten.

Außerdem ermöglicht dieses Verfahren keine zerstörungsfreie Analyse von Zellen, da die Zellen mit den Antikörpern markiert und somit nicht mehr "natürlich" sind, sodass diese nicht für weitere Untersuchungen oder Anwendungen zur Verfügung stehen.

Den Erfindern ist ferner aufgefallen, dass bei den bestehenden RT-DC-Systemen ein Nachteil darin besteht, dass die mikrofluidischen Kanäle immer auf die Zellgröße angepasst werden müssen. So muss der Kanal immer leicht größer als der Durchmesser der größten zu untersuchenden Zelle sein: Ist der Durchmesser des Kanals zu klein, bleibt die Zelle "hängen", ist der Durchmesser des Kanals hingegen deutlich größer als der Durchmesser der Zelle, wirken aufgrund der Hydrodynamik keine nennenswerten Scherkräfte auf die Zelle ein, d.h. diese wird nicht oder nur unwesentlich deformiert. Wenn eine Zelle "hängen bleibt", ist dies ein erheblicher Nachteil, da man dadurch in vielen Fällen den Rest der Probe nicht mehr verwenden kann: Oft ist das gesamte Probenvolumen nur wenige µl, und da dieses Probenvolumen den gesamten Chip füllt und der Chip bei einer hängen bleibenden Zelle ausgetauscht werden muss, wird somit das gesamte Probenvolumen (oder ein erheblicher Teil davon) weggeworfen. Da die Proben oft nur schwer beschafft werden können, ist dies ein großes Problem und ein nicht zu vernachlässigender Kostenfaktor.

Dies führt im Stand der Technik im Hinblick auf RT-DC dazu, dass die mikrofluidischen Chips gegebenenfalls während einer Messreihe ausgetauscht werden müssen, was zu Verunreinigungen und Einbußen im Probendurchsatz führt. Außerdem sind Messungen, die bei RT-DC in Chips mit verschiedenen Größen durchgeführt wurden, nicht immer vergleichbar. Dies liegt daran, dass die Kräfte auf die Zellen mit der Größe der Zellen und dem Querschnitt des Kanals skaliert. Insofern hat man zwei Skalierungsgrößen, die unabhängig voneinander eingestellt werden können. Wird eine dieser Größen (z.B. die Größe des Kanals) verändert, sind somit die Ergebnisse nicht vergleichbar. Außerdem sind derzeit einige Zellen nicht vermessbar, da die Kanäle nicht klein genug gemacht werden können.

Des Weiteren ist die Kanalgröße durch den Herstellungsprozess der mikrofluidischen Chips limitiert. Konkret kann beispielsweise das Kunststoffmaterial, aus dem der Kanal besteht, nur bis zu einer minimalen Höhe von einigen Mikrometern stabile Kanalgeometrien gewährleisten. Dies liegt daran, dass diese Chips derzeit durch Softlithographie hergestellt werden. Diese Verfahren sind sehr kostenintensiv und sind in O. Otto et al., Real-Time Deformability Cytometry: On the Fly Mechanical Phenotyping. Nature Methodes, 12, 199-202 (2015), beschrieben. Die hohen Kosten verhindern es, das Verfahren bei einer anwendungsorientierten Forschung effizient einzusetzen. Ein anderes Herstellungsverfahren wäre durch Spritzguss, wobei aber eine Negativform vorliegen muss. Hierdurch ist man in der minimalen Größe der Formen, die auszubilden sind, begrenzt. Außerdem ist ein solches Verfahren initial tendenziell teuer, da die Negativform sehr teuer ist.

Um eine Kombination von Fluoreszenzmessung, Sortierung und mechanischer Analyse zu erreichen ist eine Verwendung der mikrofluidischen Chips aus RT-DC alleine nicht ausreichend, da die Proben die oben beschriebenen Schwierigkeiten (Verstopfung und Anpassung der Chipgröße an die Samplegröße) auftreten könnten. Demgemäß ist es schwer, RT-DC in das von Dittrich und Göhde entwickelte Durchflusszytometer nach DE 1 815 352 zu integrieren.

Die US 2017/0003273 A1 offenbart Merkmale, die unter den Oberbegriff des Anspruchs 1 fallen.

### Darstellung der Erfindung

Die Erfindung zielt darauf ab, die genannten Probleme und insbesondere die Probleme, die bei bestehenden RT-DC-Systemen genannt wurden, zu lindern. Insbesondere ist es eine Aufgabe der Erfindung, die Messungen an Kanalgeometrien sowie Messsysteme und Probenvorgaben anzupassen, ohne die Messapparatur direkt und physisch in erheblichem Maße umzugestalten und umbauen zu müssen. Außerdem wird beabsichtigt, zumindest in einigen Ausführungsformen eine Charakterisierung und ggf. Sortierung in einem Durchflusszytometer zu ermöglichen. So haben in Durchflusszytometern die verwendeten Küvetten typischerweise einen Durchmesser von mehreren 100 µm, was für eine mechanische Zellmessung zu groß ist, da die auf die Zellen ausgeübten Kräfte viel zu gering wären. Es ist ein Ziel der vorliegenden Erfindung, auch mit Küvetten mit solchen Durchmessern Verformungen von Zellen verursachen zu können und Messungen von mechanischen Eigenschaften dieser Zellen durchführen zu können.

Die Erfindung wird durch Anspruch 1 definiert. Bevorzugte Ausführungsformen werden durch die abhängigen Ansprüche definiert.

Ein erfindungsgemäßes Verfahren ist ein Verfahren zum Verformen von deformierbaren Körpern, welche z. B. Tröpfchen oder Zellen sein können. Bei einem deformierbaren Körper handelt es sich um einen Körper, der in einem fließenden Fluid (bevorzugt: Flüssigkeit) transportiert werden kann und der durch die bei einem solchen Fluss auftretenden Scherkräfte oder durch die an der Grenzschicht zwischen den Fluiden auftretenden Kräfte verformt werden kann.

Das erfindungsgemäße Verfahren weist einen Schritt des Zuführens eines Probenfluids in einen Kanal auf, so dass ein laminarer Fluss entsteht. Bei diesem Probenfluid handelt es sich um ein Fluid, welches deformierbare Körper enthält und diese transportiert. Diese deformierbaren Körper sind in dem Probenfluid in Form einer Suspension bzw. Aufschlämmung enthalten und werden somit mit dem Probenfluid mittransportiert, wenn dieses durch den Kanal fließt.

Bei dem Kanal handelt es sich bevorzugt um einen mikrofluidischen Kanal. Dies ist ein Kanal mit einem Durchmesser von deutlich weniger als 1 mm und insbesondere einen Kanaldurchmesser im Bereich von 100 nm bis 500 um. Unter einem laminaren Fluss wird ein Fluss verstanden, bei dem keine Turbulenzen auftreten. Ein solcher Fluss kann dadurch definiert werden, dass die Reynolds-Zahl deutlich kleiner als 1000 ist.

Es wird ferner erfindungsgemäß ein Hüllfluid in den Kanal eingeführt, in dem das Probenfluid fließt. Hierbei wird dieses Hüllfluid so eingeführt, dass ein laminarer Fluss dieses Hüllfluids entsteht und sodass das Hüllfluid an das Probenfluid in einem Angrenzungsbereich des mikrofluidischen Kanals direkt angrenzt. Unter "direkt angrenzen" wird verstanden, dass es zwischen diesen beiden Flüssen keine physische Trennung gibt, d. h. dass die beiden Flüsse ohne Barriere aneinander angrenzen und prinzipiell auch ineinander übergehen könnten. Bei dem Angrenzungsbereich kann es sich um die Gesamtlänge des Kanals handeln, es reicht jedoch aus, wenn die beiden Fluide nur in einem Teilbereich dieses Kanals aneinander direkt angrenzen, während sie in anderen Bereichen voneinander getrennt sind.

Bei dem Hüllfluid kann es sich um ein beliebiges anderes Fluid (bevorzugt: Flüssigkeit) als das Probenfluid handeln, wobei jedoch später die beiden Fluide noch genauer definiert werden. Bevorzugt ist das Hüllfluid an zwei Seiten des Probenfluids vorgesehen, wobei bevorzugt dieser Fluss des Hüllfluids symmetrisch in Bezug auf das Probenfluid ist. Jedoch reicht es aus, wenn das Hüllfluid an einer Seite des Probenfluids ist. Eine symmetrische Anordnung, insbesondere eine Anordnung, bei welcher das Hüllfluid den Fluss des Probenfluids umgibt, führt zu einer symmetrischen Geometrie, die somit auch zu einer symmetrischen Verformung der Zellen und somit zu einer leichteren und besseren Analyse führt.

Mehr im Hinblick auf Physik können Zellen bzw. deformierbare Körper allgemein als elastische Objekte modelliert werden, die sich aufgrund von Scher- und Normalkräften in engen Mikrokanälen verformen lassen. Für Experimente im RT-DC ist eine umfassende Darstellung der auf sie wirkenden Kräfte in A. Mietke et al., Extracting Cell Stiffness from Real-Time Deformability Cytometry: Theory and Experiment, Biophysical Journal 109, 2023-2036 (2015) beschrieben.

Die wichtigsten Parameter, die den Grad der Verformung bestimmen, sind die Elastizität und Viskosität (Viskoelastizität) der Zellen, die Oberflächenspannungen der verwendeten Zellen sowie die Viskositäten und die Oberflächenspannung des Hüll- und Probenfluids, die maßgeblich für die Skalierung des Geschwindigkeitsprofils und damit für die Scher- und Normalkräfte sind. In RT-DC wird die Verformung der Zellen (Deformation D) beschrieben durch D=1-(2√(πA))/P. Hierbei ist A die Querschnittsfläche (Projektionsfläche) und P der Umfang der Zellen. Diese Definition der Verformung als "circularity" ist jedoch nur ein Beispiel, und andere Definitionen der Verformung können auch verwendet werden. Andere Definitionen der Verformung wären das Flächenträgheitsmoment und das Achsenverhältnis.

Erfindungsgemäß ist die dynamische Viskosität des Hüllfluids größer als die dynamische Viskosität des Probenfluids. Unter der dynamischen Viskosität wird hierbei die Viskosität verstanden, wie sie bei den in den beiden Fluiden im Angrenzungsbereich des Kanals auftretenden Scherraten auftritt. Dies kann dadurch realisiert werden, dass das Hüllfluid entweder scherverdickend ist oder aber eine newtonsche Flüssigkeit mit einer hohen Viskosität ist, während das Probenfluid scherverdünnend ist oder aber eine newtonsche Flüssigkeit mit einer geringen Viskosität ist. Vor allem eine scherverdünnende Flüssigkeit als Probenfluid wird bevorzugt, da sie zu einem besonders vorteilhaften Verhalten der Flüssigkeiten führt. Bei all dem ist jedoch wichtig, dass das Probenfluid immer eine niedrigere dynamische Viskosität bei den auftretenden Scherraten als das Hüllfluid bei den auftretenden Scherraten hat. Hierdurch wird sichergestellt, dass das Hüllfluid einen virtuellen Kanal für das Probenfluid bildet, wie dies später im Detail beschrieben werden wird. Die hierbei typischerweise auftretenden Scherraten liegen im Bereich von 100 1/s bis 10.000 1/s, und es wird bevorzugt, dass das Hüllfluid und das Probenfluid die genannten Bedingungen für die Viskositäten für alle Scherraten in diesem Bereich erfüllen. Es ist auch möglich, dass das Hüllfluid und das Probenfluid bei kleinen Scherraten die gleiche Viskosität aufweisen und das Probenfluid bei höheren Scherraten Scherverdünnung aufweist.

Außerdem ist bevorzugt, dass die durchschnittliche Flussgeschwindigkeit des Probenfluids größer ist als diejenige des Hüllfluids. Unter der durchschnittlichen Flussgeschwindigkeit des Probenfluids wird verstanden, dass die Flussgeschwindigkeit als arithmetisches Mittel der Flussgeschwindigkeit in einem Querschnitt senkrecht zur Flussrichtung des Fluids in dem mikrofluidischen Kanal bestimmt wird. Die Viskosität des Hüllfluids und des Probenfluids bezieht sich jeweils auf die Viskosität bei Raumtemperatur wie sie mit einem Scherrheometer von Anton Paar MC302 gemessen wird, bei dem ein Kegel-Platte System mit 50mm Durchmessen und 2 Grad Winkel sowie mit einem Zylindersystem verwendet wird.

Durch das Zuführen von zwei verschiedenen Fluiden kann im Gegensatz zu der in der WO 2015/024690 A1 beschriebenen Technik der Kanal "virtuell" verkleinert werden. Derjenige Teil des Kanals, in dem das Probenfluid fließt, bildet den eigentlichen Kanal, d. h. denjenigen Teil des Kanals, in dem sich die deformierbaren Körper im parabolischen Flussprofil aufgrund des laminaren Flusses verformen (Li et al., J. Fluids Eng. 2011, 111202) . Im Gegensatz dazu bildet das Hüllfluid sozusagen einen Teil der Begrenzung des mikrofluidischen Kanals, was die Flusseigenschaften des Probenfluids angeht. Anders gesagt ist dieses, da es bei der vorliegenden Scherrate eine höhere dynamische Viskosität hat als das Probenfluid, vergleichsweise "fest" in Bezug auf das Probenfluid, weshalb es wie die Wand des mikrofluidischen Kanals in der WO 2015/024690 A1 wirkt. Dieses Verfahren des virtuellen Änderns der Größe des Kanals wird als "Virtual Channel Resizing" bezeichnet.

Hierbei ist es von Vorteil, dass der virtuelle Kanal, der durch das Probenfluid 10 gebildet wird, mikrofluidisch ist, d.h. dass er die Definition eines mikrofluidischen Kanals erfüllt. Der äußere Kanal 12 kann jedoch deutlich größer sein und muss nicht mikrofluidisch sein. Z.B. könnte dieser eine Küvette sein.

Demgemäß kann durch eine Regelung der jeweiligen Flussraten des Hüllfluids und des Probenfluids der Anteil des mikrofluidischen Kanals, in dem das Hüllfluid bzw. das Probenfluid fließt, gesteuert werden. Dies führt dann dazu, dass man die Kanalbreite des "virtuellen" mikrofluidischen Kanals (der durch das Probenfluid definiert wird) virtuell einstellen kann, ohne jedoch die physische Breite des eigentlichen Kanals separat einzustellen. Hierdurch kann man mit dem gleichen Kanal verschiedene Zellen untersuchen, ohne dass man jeweils für bestimmte Zelltypen verschiedene Kanäle verwenden muss.

Um das gewünschte Strömungsprofil zu erhalten, können mit Hilfe von Li et al., J. Fluids Eng. 2011, 111202, Gleichung [20], die Viskositäten, die Flussraten und die Kanalgeometrie eingestellt werden.

Erfindungsgemäß liegt die dynamische Viskosität des Hüllfluids im Bereich von 50 mPa s bis 250 mPa s und liegt die Viskosität des Probenfluids im Bereich von 5 mPa s bis 50 mPa s. Entsprechende Bereiche haben sich als besonders vorteilhaft erwiesen. Die dynamischen Viskositäten liegen in diesen Scherratenbereichen in dem Angrenzungsbereich und bevorzugt über den gesamten Scherratenbereich von 100 1/s bis 10.000 1/s.

Bevorzugt liegt die durchschnittliche Flussgeschwindigkeit des Probenfluids im Bereich von 0,1 cm/s bis 1 m/s und/oder liegt die durchschnittliche Flussgeschwindigkeit des Hüllfluids im Bereich von 0,1 cm/s bis 1 m/s, jedoch auf jeden Fall unterhalb der durchschnittlichen Flussgeschwindigkeit des Probenfluids. Entsprechende bevorzugte Flussgeschwindigkeiten haben sich in Versuchen als besonders vorteilhaft erwiesen. Bevorzugt wird aus den gleichen Gründen auch, dass die Flussraten (Volumenstrom) von Probenfluid zum Hüllfluid im Bereich von 1:1 bis 20:1 liegen. Dies wurde experimentell gezeigt, allerdings sind auch Flussraten (Volumenstrom) von Probenfluid zum Hüllfluid von 1:1 bis 1:20 möglich, je nach Kanalgeometrie.

Es wird weiterhin bevorzugt, dass das Probenfluid eine scherverdünnende Flüssigkeit ist, welche die deformierbaren Körper enthält. Unter Scherverdünnung wird verstanden, dass die Viskosität des Probenfluids bei dem Aufbringen einer Scherkraft geringer wird. Hierdurch kann das Probenfluid schneller fließen, da es einen geringeren Widerstand ausübt. Dies ist für das Implementieren des erfindungsgemäßen Verfahrens von Vorteil.

Es wird weiterhin bevorzugt, dass das Probenfluid und das Hüllfluid aus Flüssigkeiten bestehen, die sich zumindest in der Zeitskala, in der die deformierbaren Körper den Angrenzungsbereich durchqueren, nicht oder nur unwesentlich vermischen. Insbesondere könnte dieses Merkmal dadurch realisiert werden, dass die eine Flüssigkeit polar ist, während die andere Flüssigkeit apolar ist. Durch das Verwenden entsprechender Flüssigkeiten wird erleichtert, die Flüssigkeiten zu trennen, was zu einer einfacheren Hydrodynamik und somit einer besseren Analysierbarkeit der Ergebnisse führt.

Weiterhin wird bevorzugt, dass das Hüllfluid eine newtonsche Flüssigkeit ist. Unter einer newtonschen Flüssigkeit wird eine Flüssigkeit verstanden, deren Viskosität sich nicht oder nur unwesentlich bei verschiedenen Scherraten verändert. Durch das Verwenden einer solchen newtonschen Flüssigkeit kann sichergestellt werden, dass die mechanischen Eigenschaften des Hüllfluids sich nur geringfügig, wenn überhaupt, verändern. Auch mit einem solchen Verfahren können vergleichsweise gute und zuverlässige Ergebnisse erzielt werden. Jedoch kann das Hüllfluid auch scherverdickend sein, was den genannten Effekt, einen virtuellen Kanal zu bilden, weiter verstärkt.

Es wird weiterhin bevorzugt, dass das Verfahren zum Bestimmen der mechanischen Eigenschaften von verformbaren Körpern, insbesondere Zellen eingesetzt wird. Hierbei wird die Verformung der deformierbaren Körper mittels eines optischen Verfahrens bevorzugt gemessen. Insbesondere kann hierbei das Verfahren verwendet werden, wie es in der WO 2015/024690 A1 verwendet wird. Dadurch können die mechanischen Eigenschaften von Zellen effizient bestimmt werden. In der WO 2015/024690 A1 wird auch beschrieben, wie die optischen Daten ausgewertet werden können, um mechanische Eigenschaften zu bestimmen.

Eine weitere Anwendung des Verfahrens besteht darin, dass man das erfindungsgemäße Verfahren mit bereits bestehenden Technologien zur Analyse von Zellen im Hinblick auf deren Zellbiologie kombinieren kann. So kann man z.B. mit Hilfe von fluoreszierenden Farbstoffen Organellen von Zellen oder z.B. Proteine in deren Zytoskelett markieren (z.B. Aktin). Wenn man nun die Zellen mit dem erfindungsgemäßen Verfahren verformt, kann man beobachten, wie sich das Zytoskelett verändert oder wie sich die Organellen verändern. Dies ermöglicht eine komplett neue Analyse von Zellen.

Insbesondere kann eine solche Analyse mit Hilfe von Durchflusszytometrie durchgeführt werden (die z.B. unter der Marke "FACS" bekannt ist). Ein solches Verfahren ist z.B. in A. Adan, Flow cytometry: basic principles and applications, Crit. Rev. Biotechnol., 37, 163-176, 2017 beschrieben.

Prinzipiell wäre es mit RT-DC möglich, Chips in ein Durchflusszytometer einzubauen. Jedoch wäre es wahrscheinlich, dass diese verstopfen. Außerdem wäre es unpraktisch, je nach Zellgröße verschiedene Chips verwenden zu müssen. Da man bei der vorliegenden Technologie nicht auf mikrofluidische Kanäle beschränkt ist, besteht eine solche Verstopfungsgefahr nicht. Es ist daher realistisch möglich, ein solches Verfahren umzusetzen.

Weiterhin kann das erfindungsgemäße Verfahren zum Sortieren von verformbaren Körpern, insbesondere Zellen verwendet werden. Prinzipiell gibt es zwei Sortierverfahren: aktives und passives Sortieren. Bei dem passiven Sortieren werden die Eigenschaften von Zellen oder allgemein verformbaren Körpern ausgenutzt, um diese zu sortieren. So kann man z.B. die Trägheitseigenschaften von verformbaren Körpern einsetzen, um diese zu sortieren. Die Körper trennen sich sozusagen durch das Einsetzen einer bestimmten Filtervorrichtung von selbst, als ohne externes Einwirken, in zwei Populationen auf. Ein einfaches Beispiel aus dem Alltag für ein passives Sortieren wäre ein Sieb. Auch hier bedarf es keiner externen Einwirkung, um die großen Partikel von den kleinen Partikeln zu trennen: Die einen fallen durch das Sieb hindurch, während die anderen in diesem hängen bleiben. Ein Problem hierbei ist jedoch, dass man für diese Sortierung die Eigenschaften der Körper vorher wissen muss, was nicht immer der Fall ist. Beim Beispiel des Siebs muss man z.B. die typischen Größen der Partikel wissen.

Bei einem aktiven Sortieren bewegt man die zu sortierenden Körper aktiv in die eine oder andere Population. Ein Beispiel aus dem Alltag wäre z.B. das manuelle Sortieren von Gegenständen aufgrund der Größe. Im Gegensatz zu dem genannten Beispiel des Siebs ist hier ein externes Handeln nötig. Bei verformbaren Körpern und insbesondere Zellen ist jedoch hier bei RT-DC eine komplizierte Kopplung aus Mikrofluidik und einer Steuerungselektronik nötig, die sehr aufwendig sind und die nicht oder nur schlecht skalierbar ist.

Da das vorliegende System auf eine Mikrofluidik verzichten kann, wird diese Komplikation umgangen, weshalb es für eine Zellsortierung bzw. Sortierung von verformbaren Körpern eingesetzt werden kann. Anders gesagt kann, da das vorliegende System eine Integration der mechanischen Zellvermessung in ein Durchflusszytometer erlaubt, dessen Sortiereinheit (z.B. basierend auf Piezoelementen) direkt und ohne größere Modifikationen genutzt werden. Eine solche Sortiereinheit übt einen Impuls auf den jeweiligen verformbaren Körper bzw. Zelle aus, so dass sich dieser Körper/diese Zelle in ein jeweils anderes Reservoir bewegt. Insbesondere kann ein Durchflusszytometer (FACS) gut für ein aktives Sortieren von Zellen verwendet werden.

Bei gegebenen Kanalgeometrien und Materialien für Probenfluid und Hüllfluid wird die Breite des Stroms des Probenfluids über die Wahl des Flussratenverhältnisses eingestellt. Zusätzlich hängt die Breite des Probenfluids auch von der Gesamtflussrate ab. Hierbei wird besonders bevorzugt, dass vor Beginn des Verformens bzw. des Messens die Gesamtflussrate von Probenfluid und Hüllfluid (d. h. der Gesamtfluss an Flüssigkeit bzw. Fluid pro Zeit, also der Volumenstrom) von einem Anfangswert zu einem Endwert bzw. Zielwert vergrößert werden. Den Erfindern ist aufgefallen, dass die Fluide oft ein Hystereseverhalten an den Tag legen, bei dem sich die relative Breite des Stroms des Probenfluids in Bezug auf den Kanal über einen großen Bereich der Flussraten bei einem Erhöhen der Flussrate konstant bleibt, während ein solches Verhalten bei einer abnehmenden Flussrate nicht in diesem Maße auftritt. Da es allgemein von Vorteil ist, bei verschiedenen Flussraten eine relative Breite des Stroms des Probenfluids in Bezug auf den Kanal konstant zu halten, und da ein entsprechendes Verhalten nur bei einer zunehmenden absoluten Flussrate bzw. Gesamtflussrate beobachtet wird, ist es von Vorteil, die Gesamtflussrate zu erhöhen, nicht aber sie herunterzufahren.

Weiterhin wird offenbart, dass eine Vorrichtung bereitgestellt wird, die zum Durchführen des Verfahrens nach einem der vorhergehenden Ansprüche ausgestaltet ist. Eine solche Vorrichtung weist insbesondere eine Steuerungseinrichtung und Zufuhren für ein Hüllfluid bzw. ein Probenfluid auf, welche die entsprechenden Merkmale aufweisen.

### Kurze Beschreibungen der Zeichnungen

Figur 1 ist ein Foto eines mikrofluidischen Kanals zur Durchführung eines erfindungsgemäßen Verfahrens. Ein laminarer Strom aus Probenfluid wird durch zwei ebenfalls laminare Ströme aus Hüllfluid auf einen engen Kanal fokussiert.
Figur 2 ist eine Draufsicht eines Abschnitts des mikrofluidischen Kanal. Hellfeld-Aufnahme mit Durchlicht mit Fokus auf den engen Kanal 12.
Figur 3 zeigt das Verhalten der relativen Breite des Stroms des Probenfluids zu der Gesamtbreite des Kanals in Abhängigkeit von der Flussrate. Die Insets zeigen Bilder des Kanals bei verschiedenen Flussraten.
Figur 4 zeigt das Verformungsverhalten eines Leukozyten gemäß dem Stand der Technik.
Figur 5 zeigt das Verformungsverhalten eines Leukozyten gemäß dem erfindungsgemäßen Verfahren.
Figur 6(a) zeigt ein Scatterplot für Leukozyten gemäß dem Stand der Technik.
Figur 6(b) zeigt einen Scatterplot eines Leukozyten gemäß dem erfindungsgemäßen Verfahren.
Figur 7 zeigt Messergebnisse zu den dynamischen Viskositäten zu den verwendeten Flüssigkeiten.

### Detaillierte Beschreibungen der Zeichnungen

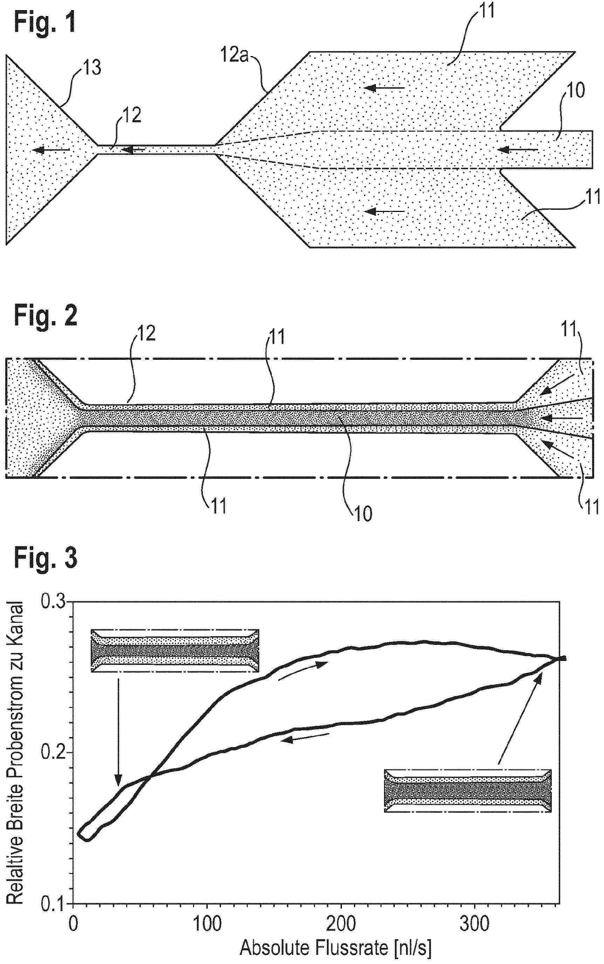

Figur 1 zeigt ein Foto des verwendeten mikrofluidischen Chips. Der mikrofluidische Kanal 12 weist einen Einlass 12a auf, in den Hüllfluide 11 von zwei Seiten eines Probenfluids 10 zugeführt wird. Die Flussrichtungen innerhalb dieses Kanals sind jeweils durch Pfeile dargestellt. Wie aus Figur 1 ersichtlich ist, umgeben die zwei Hüllfluide 11 das Probenfluid 10 symmetrisch. Nachdem diese Hüllfluide 11 den mikrofluidischen Kanal 12 durchquert haben, treten sie aus dem Auslass 13 aus.

Eine detaillierte Ansicht des mikrofluidischen Kanals 12 kann in Figur 2 gesehen werden. Wie hier deutlich zu erkennen ist, ist der Fluss des Probenfluids 10 auch innerhalb des mikrofluidischen Kanals 12 deutlich von den beiden seitlichen Strömen des Hüllfluids 11 getrennt. Dies ist sogar in einer Hellfeldaufahme wie in Fig. 2 gezeigt sichtbar. Insofern fließt innerhalb des mikrofluidischen Kanals 12 der Strom des Probenfluids 10 in einem virtuellen Kanal, der durch das Hüllfluid 11 begrenzt wird.

In Hinblick auf physikalische Grundlagen ist zu bemerken, dass in laminaren Flusssystemen, die durch kleine Reynolds-Zahlen (Reynolds-Zahl kleiner als 1000) charakterisiert sind, sich entlang eines Kanals eine Strömung in Fließrichtung ausbildet, die senkrecht zur Fließrichtung ein parabelförmiges Geschwindigkeitsprofil hat. Insbesondere gilt an den Kanalrändern die Bedingung, dass die Geschwindigkeit der Moleküle 0 zu sein hat. Gleichzeitig wird die höchste Geschwindigkeit dort erreicht, wo die Moleküle am wenigsten von den Rändern oder weiteren Strömen in ihrem Fluss gestört werden. Für laminar durchströmte Kanäle ist dies in deren Mitte. An der Grenze zwischen den beiden Fluiden ist die Geschwindigkeit dieser Fluide ungleich null.

Wie in Figur 1 dargestellt, fließen die Hüll- und Probenfluide 11 und 10 von rechts nach links, wobei die Hüllfluide 11 das Probenfluid 10 fokussieren. Diese Fluide 10, 11 fließen laminar durch den mikrofluidischen Kanal 12, der in dem in Figur 2 gezeigten Beispiel eine Abmessung von 40 um x 40 um im Querschnitt senkrecht zur Flussrichtung hat. Innerhalb des engen virtuellen mikrofluidischen Kanals 12, durch den das Probenfluid 10 fließt, erhöht sich die Geschwindigkeit des Stroms des Probenfluids 10 auf etwa 50 cm pro Sekunde in der Kanalmitte. Die Breite des virtuellen Kanals, durch den das Probenfluid 10 fließt, hängt bei gegebener Kanalgröße und Viskositäten von den Flussraten ab, die durch Spritzenpumpen, welche den Strom des Hüllfluids 11 und des Probenfluids 10 bereitstellen, übertragen wird. Spritzenpumpen sind vorteilhaft, weil sie eine präzise Ansteuerung der Flussmengen mit einer Genauigkeit von nl/s ermöglichen. In einem RT-DC-Experiment wird das Verhältnis zwischen den Flussraten im Hüllfluid und Probenfluid so eingestellt, dass die Zellen mittig den mikrofluidischen Kanal 12 durchströmen. Beim Virtual Channel Resizing besteht das Hüllfluid in der vorliegenden Ausführungsform aus einer Polymerlösung (z. B. 100 mMol Polyethylenglycol 8000 in einer phosphathaltigen Pufferlösung), die deutlich viskoser ist als das Probenfluid. Wie gezeigt, mischen sich die Ströme nur wenig, was an den klaren Kanten in Figur 2 deutlich wird. Dies wurde durch Finite-Elemente-Simulationen bestätigt. Aufgrund der Durchflusszeit der beiden Fluide fand eine Diffusion nicht oder nur in unwesentlichem Maße statt.

In der vorliegenden Ausführungsform findet das Virtual Channel Resizing auf folgende Weise statt: Die Zusammensetzung der Hüllfluide 11 wird so gewählt, dass sie eine gegenüber dem Probenfluid 10 deutlich erhöhte Viskosität haben, die jedoch nur schwach von der Scherrate abhängig ist.

Die ersten Experimente wurden mit Polyethylenglycol (PEG) 8000 durchgeführt, das in einer Konzentration von 100 mMol komplett in PBS (phosphatgepufferte Salzlösung) gelöst wurde. Hierbei wurde PBS verwendet, welches kein Calcium und Magnesium in nennenswerten Mengen enthält. Prinzipiell kann jedoch auch eine andere Flüssigkeit verwendet werden. löst. Die Lösung hat eine dynamische Viskosität von ungefähr 235 mPa s (bei einer Scherrate zwischen 1 1/s - 10.000 1/s). Diese Viskosität wurde mit einem Rheometer von Anton-Paar MC302 mit einem Kegel-Platte System mit 50mm Durchmessen und 2 Grad Winkel sowie mit einem Zylindersystem gemessen. Die Lösungen des Probenstroms wurden mit einem Kugel-Roll-Viskosimeter (Anton-Paar, Lovis2000-DMA) gemessen. Demgemäß werden die Flussgeschwindigkeiten am Kanalrand mehr als 20-fach verringert. Eine analytische Beschreibung dieses Verhaltens findet sich in J. Li, P. S. Sheeran, C. Kleinstreuer, Analysis of Multi-Layer Immiscible Fluid Flow in a Microchannel, J. Fluids Eng. 133, 111202 (2011).

Das parabelförmige Profil des Stroms des Probenfluids 10 bildet sich über einen virtuell kleineren Kanal aus, der jetzt durch die Grenzfläche zwischen dem Probenfluid 10 mit den Hüllfluiden 11 gegeben ist. Dazu werden im Strom des Probenfluids im Vergleich zum RT-DC nach WO 2015/024690 A1 die Scherraten-Änderungen senkrecht zur Fließrichtung größer. Dies führt zu größeren Scher- und Normalkräften im Probenfluid 10 als dies bei den bisherigen RT-DC-Versuchen der Fall war. Zwar könnte man auch hier die Flussraten oder die Chips verändern, was aber zu einer verringerten Vergleichbarkeit der Ergebnisse führen würde. Zusätzlich werden Nachteile wie beispielsweise häufiger Chipwechsel und/oder Verstopfen der Kanäle vermieden.

Aus den genannten Betrachtungen ergibt sich als bevorzugte Bedingungen für die Zusammensetzung der Hüllfluide 11 Folgendes: Es ist von Vorteil, dass die Zusammensetzung der Materialien und Lösungen für das Probenfluid 10 und Hüllfluide 11 so gewählt sind, dass diese Fluide 10, 11 sich in der Zeitskala, in der die deformierbaren Objekte sich verformen und durch den Kanal fließen, nicht aufgrund der Diffusion mischen.

Ferner ist es von Vorteil, dass das Material/die Lösung des Hüllfluids 11 eine höhere Viskosität als das Material/die Lösung des Probenfluids 10 hat. Es ist jedoch auch denkbar, dass das Hüllfluid und das Probenfluid bei kleinen Scherraten die gleiche Viskosität haben, jedoch das Probenfluid scherverdünnend ist. Die Viskosität des Hüllfluids 11 sollte möglichst wenig von der Scherrate abhängen (also newtonsch sein) oder aber scherverdickend sein. So ist für PEG 8000 (100 mMol) in PBS die dynamische Viskosität (gemessen mit einem Rheometer) ungefähr 235 mPa s über einen Scherratenbereich von 1 pro Sekunde bis 10.000 pro Sekunde.

Es ist ferner von Vorteil, dass das Material/die Lösung des Probenfluids scherverdünnend ist: Für Methylcellulose (0,5 %) in PBS folgt die dynamische Viskosität einem Power-Law mit einem Exponenten von 1 bis 0,677 (Herold, ArXiv 2017, https://arxiv.org/ftp/arxiv/papers/1704/1704.00572.pdf) .

Erfolgreiche Realisierungen von Virtual Channel Resizing lassen sich mit unterschiedlichen Bedingungen erzielen. Experimentell wurde das mit den in der folgenden Tabelle gelisteten Zusammensetzungen von Proben- und Hüllfluid gezeigt:

| Erfolgreiche Realisierung | **Material Probenfluid** | **Material Hüllfluid** |
|---|---|---|
| 1 | Methylcellulose (0.5% w/v) in PBS, dynamische Viskosität 14mPa s, scherverdünnend, 285 mOsm | Polyethylenglycol 8000 (100mM) in PBS, dynamische Viskosität 235mPa s, nahezu newtonisch, > 2800 mOsm |
| 2 | Methylcellulose (0.7% w/v) in PBS, dynamische Viskosität 21mPa s, scherverdünnend, 289 mOsm | Polyethylenglycol 8000 (100mM) in PBS, dynamische Viskosität 235mPa s, nahezu newtonisch, > 2800 mOsm |
| 3 | Methylcellulose (0.5% w/v) in PBS, dynamische Viskosität 14mPa s, scherverdünnend, 285 mOsm | Polyethylenglycol 8000 (20mM) in PBS, dynamische Viskosität 18mPa s, nahezu newtonisch, etwa 600 mOsm |

| | | |
|---|---|---|
| 4 | Methylcellulose (0.5% w/v) in PBS, dynamische Viskosität 14mPa s, scherverdünnend, 285 mOsm | Polyethylenglycol 6000 (100mM) in PBS, dynamische Viskosität 32mPa s, nahezu newtonisch, > 1400 mOsm |

Für alle Realisierungen, die in der Tabelle aufgeführt sind, bilden sich im Bereich von Gesamtflussraten von 3 bis 400 nl/s eine wohldefinierte Entmischungsgrenze an den Grenzen zwischen Proben- und Hüllfluid unabhängig von den verwendeten Flussraten aus. Diese ist mittels Hellfeldmikroskopie deutlich zu sehen, wie dies in Figuren 2, 3 und 5 gezeigt ist, bei Verwendung von Phasenkontrastmikroskopie sogar deutlich verstärkt.

In der Erfindung wurden die Flussraten des Probenfluids 10 und der Hüllfluide 11 unabhängig voneinander durch Pumpen eingestellt. Dies kann auch durch Druck, Elektroosmose, Kapillarkräfte und Hydrostatik erzielt werden. Über das erfindungsgemäße Virtual Channel Resizing lässt sich die Breite des Stroms des Probenfluids 10 einstellen, was sich direkt auf dessen parabolisches Flussprofil auswirkt, wie dies vorher erwähnt wurde.

Hierzu gibt es zwei Möglichkeiten. Zum einen kann man, wie im konventionellen RT-DC-Experiment nach WO 2015/024690 A1 das Verhältnis der Flussraten für Hüllfluide 11 und Probenfluid 10 anpassen, um die Fokussierung des Probenfluids 10 anzupassen. Wichtig ist dabei jedoch, dass das Flussprofil im mikrofluidischen Kanal 12 außerdem von der absoluten Flussrate abhängt. Ein entsprechender Effekt wird z. B. in Figur 3 gezeigt.

Zum anderen kann bei dem Virtual Channel Resizing auch die Breite des Stroms des Probenfluids 10 über die Zusammensetzung von Proben- und Hüllfluid und die absolute Kanalbreite eingestellt werden. Die Zusammensetzungen beeinflussen die Breite des Stroms über die dynamischen Viskositäten. Das Verhalten wird in Li et al., Gleichung [20] beschrieben.

In Figur 3 wird das Verhalten der relativen Breite des Stroms des Probenfluids 10 in Bezug auf die Gesamtbreite des Kanals 12 jeweils senkrecht zur Flussrichtung im mikrofluidischen Kanal 12 in Abhängigkeit von der gesamten bzw. absoluten Flussrate gezeigt. Auffallend ist hierbei, dass sich das Verhalten beim Erhöhen der Flussrate (nach rechts oben zeigender Pfeil) grundlegend von dem Verhalten beim Absenken der Flussrate (nach links unten zeigender Pfeil) unterscheidet. Insbesondere weist das Verhältnis der relativen Breite des Stroms des Probenfluids 10 zur Breite des Kanals 12 bei ansteigender Flussrate oberhalb einer gewissen Flussrate (vorliegend in etwa bei 200 nl/s) im Wesentlichen ein Plateau auf, während es bei absteigender Flussrate ein solches Plateau nicht aufweist. Das in Figur 3 gezeigte Experiment wurde mit der Zusammensetzung 1 (siehe Tabelle) in einem mikrofluidischen Kanal mit einem Querschnitt von 40 um x 40 um durchgeführt. Natürlich ist, wie von Li et al., J. Fluids Eng. 2011, 111202 nachgewiesen bzw. vorhergesagt wurde, die relative Breite abhängig von der Kantenlänge des Kanalquerschnitts.

In Figur 3 ist weiterhin zu erkennen, dass es mittels des Virtual Channel Resizing möglich ist, einen weiteren Bereich virtueller Kanalbreiten abzudecken, indem lediglich die Pumpen gezielt angesteuert werden müssen. Es ist also weder nötig, die Lösungen auszutauschen, noch müssen die Proben gewechselt werden.

Im vorliegenden Beispiel verhält sich das Flussratenverhältnis vom Strom des Probenfluids 10 zum Strom des Hüllfluids 11 für alle absoluten Flussraten, die in Figur 3 gezeigt sind, wie 2 zu 1. Die relative Breite des Stroms des Probenfluids 10 kann hierbei zwischen 14 % (bei 4 nl/s) und 27 % (bei 250 nl/s und beim Hochfahren der Pumpleistung) betragen. Die beiden Insets in Figur 3 verdeutlichen, wie sich diese Änderung optisch abbilden lässt, und zeigen Phasenkontrastbilder der jeweiligen Kanäle 12. Das Probenfluid 10 besteht aus 0,5 Gewichtsprozent Methylcellulose in PBS, das Hüllfluid 100 mM PEG8000 in PBS.

Hierdurch wird es möglich, die hydrodynamischen Bedingungen an die zu untersuchenden Zellen anzupassen, da man jeweils wie gewünscht den Kanaldurchmesser an den Durchmesser der Zellen anpassen kann. Insofern kann man einen mikrofluidischen Kanal 12 verwenden, welcher deutlich größer ist als die zu untersuchenden Zellen bzw. zu untersuchenden Körper, diesen dann jedoch virtuell verkleinern, sodass er an die zu untersuchenden Zellen bzw. Körper passt.

In Figur 3 ist ferner eine Hysterese der relativen Breite des Stroms des Probenfluids 10 bezüglich der Breite des mikrofluidischen Kanals 12 in Abhängigkeit von der Ansteuerung der Pumpen zu erkennen. Über ein großes Intervall von absoluten Flussraten ist die relative Breite des Stroms des Probenfluids 10 größer, wenn die Pumpleistung erhöht wird, als wenn sie abgesenkt wird.

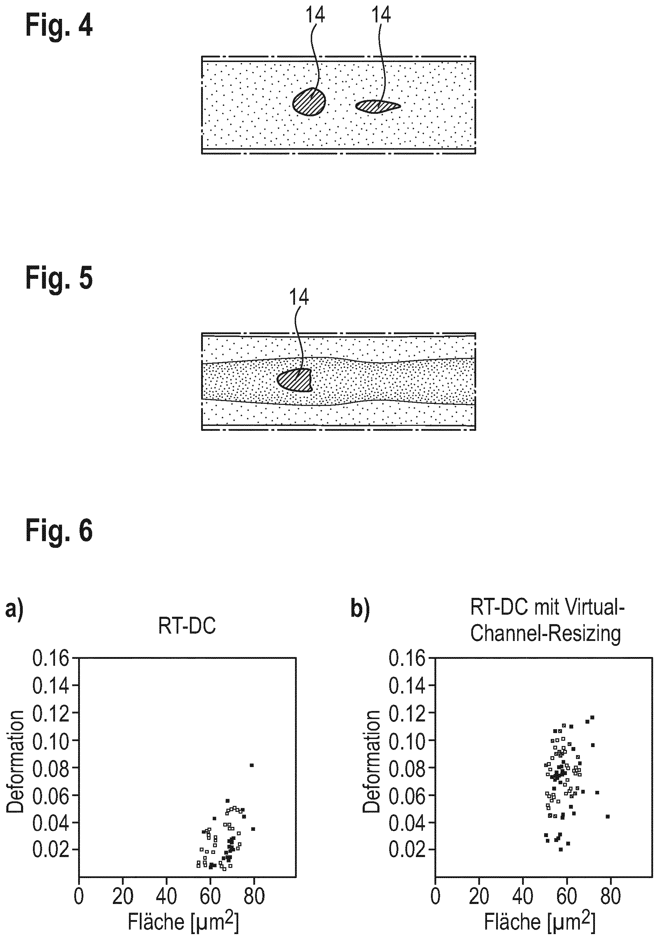

Um die Anwendung vom Virtual Channel Resizingzu verdeutlichen, wird nachfolgend in Figuren 4 bis 6 das Experiment an Leukozyten in einer Vollblutmessung dargestellt. Nach Entnahme wird das Blut in einer Citratlösung gelagert und für das RT-DC-Assay 1 zu 20 in einer Methylcellulose-PBS-Lösung für den Strom des Probenfluids 10 verdünnt. Figur 4 zeigt einen Granulozyten und einen Erythrozyten (links bzw. rechts) innerhalb eines 40 um x 40 um Kanals, wobei 0,6 Gewichtsprozent Methylcellulose in PBS verwendet wird, d. h. wobei sowohl Hüllfluid als auch Probenfluid aus der gleichen Lösung bestehen. Figur 6(a) zeigt einen Scatterplot der Leukozyten in dem gleichen Versuchsaufbau. Aus diesem Scatterplot ist ersichtlich, dass die Granulozyten sich nur wenig verformen. Figuren 5 und 6(b) zeigen ein ähnliches Experiment, wobei jedoch hier das Material des Hüllfluids PEG 8000 (100 mmol) in PBS ist. Wie aus Figur 5 deutlich zu erkennen ist, wird ein Leukozyt deutlich entlang der Flussrichtung verformt. Aus dem Scatterplot in Figur 6(b) ist zu erkennen, dass die Leukozyten eine stark erhöhte Deformation aufweisen.

Im Ergebnis ist deutlich, dass der virtuell verengte Strom des Probenfluids 10 zu einer wesentlich deutlicheren Verformung, also einer Verschiebung in der Darstellung der Figur 6 nach oben entlang der Y-Achse führt, d.h. die Zellpopulation wird in Subpopulationen aufgespaltet. Aus den Bildern der Figuren 4 und 5 wird außerdem deutlich, wie stark die Formunterschiede sind.

Aus den Beispielen wird klar, dass mittels des Virtual Channel Resizing Zellen unabhängig von der jeweiligen Größe des Kanals 12 verformt werden können. Ein relevantes Fließverhalten wird auch in Fig. 4 von Li et al. gezeigt, wobei "Case II" dieser Figur in vielen Punkten dem vorliegend verwendeten System entspricht.

Die vorliegende Erfindung ermöglicht es, die Breite des Stroms des Probenfluids 10 nur durch die Wahl der Materialien sowohl des Hüllfluids als auch des Probenfluid, des Volumenstroms der beiden Fluide (z.B. durch zugehörige Spritzenpumpen) und den Querschnitt des Kanals 12 einzustellen. Dies wird in Gleichungen [20] und [21] von Li et al. beschrieben, wobei Gleichung [21] die Scherspannungen beschreibt. Die Verformungen hängen dann ausschließlich von einer virtuellen Kanalbreite und der Viskosität, d. h. der Breite des Stroms des Probenfluids 10 ab. Somit ist die Anwendung der Erfindung nicht auf mikrofluidische Chips beschränkt, sondern kann auch in anderen Geometrien erzeugt werden. So können in Glasküvetten oder -röhren, die zwar einen größeren Durchmesser bis hin zu einigen Millimetern aufweisen, aber ebenfalls durch Probenfluid und Hüllfluid(e) befüllt werden können, mithilfe entsprechender Hüllfluide und Pumpeneinstellungen dieselben Effekte erzielt werden.

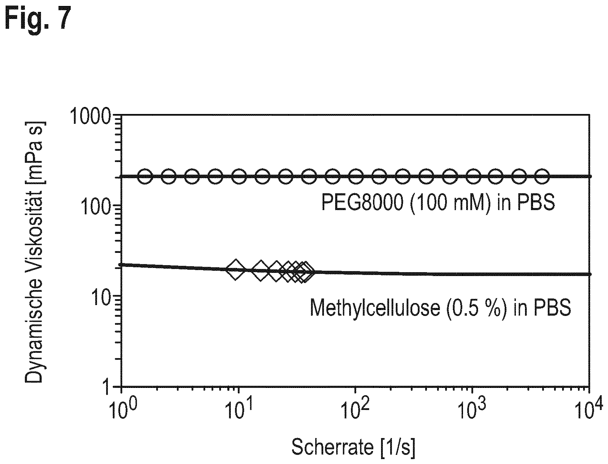

Figur 7 zeigt ferner die rheologischen Eigenschaften der verwendeten Flüssigkeiten für das Hüllfluid und das Probenfluid. Wie aus der Figur ersichtlich ist, weißt Methylcellulose in PBS ein leicht scherverdünnendes Verhalten auf, während PEG in PBS ein im Wesentlichen newtonsches Verhalten aufweist. Insbesondere ist von Relevanz, dass diese beiden Flüssigkeiten dieses Verhalten über den relevanten Scherratenbereich (1 1/s - 10.000 1/s) zeigen.

Im Rahmen der vorliegenden Erfindung sind im Hüllfluid die jeweiligen dynamischen Viskositäten größer oder gleich zu der dynamischen Viskosität des Probenfluids. Es wäre jedoch prinzipiell auch denkbar, das Viskositätsverhältnis anders herum zu wählen und immer noch Zellen bzw. Körper zu verformen, auch wenn eine solche Gestaltung derzeit als weniger vorteilhaft angesehen wird.

Ein weiterer Gesichtspunkt der Erfindung betrifft die Verwendung des erfindungsgemäßen Verfahrens zum Zuführen von Substanzen zu deformierbaren Körpern, insbesondere Zellen, wobei eine zuzuführende Substanz in dem Hüllfluid enthalten ist und wobei Teile des deformierbaren Körpers mit dem Hüllfluid in Kontakt sind, wobei durch diesen Kontakt diese Substanz in den Körper übergehen. Bevorzugt wird hierbei, dass die Fläche, über die der deformierbare Körper mit dem Hüllfluid in Kontakt steht, gesteuert wird und dass auch der Zeitraum, über den der deformierbare Körper mit dem Hüllfluid in Kontakt steht, gesteuert wird, um die Menge der zugeführten Substanz zu steuern. Ein solches Verfahren ermöglicht das gesteuerte Zuführen von Substanzen, insbesondere Medikamenten, zu deformierbaren Körpern, insbesondere Zellen.

## Patentansprüche

1. Verfahren zum Verformen von deformierbaren Körpern (14), bevorzugt Tröpfchen oder Zellen, mit folgenden Schritten:
- Zuführen eines Probenfluids (10) in einen Kanal (12), so dass ein laminarer Fluss des Probenfluids entsteht, wobei das Probenfluid (10) deformierbare Körper transportiert,
- Zuführen eines Hüllfluids (11) in den Kanal (12), so dass ein laminarer Fluss des Hüllfluids (11) entsteht und so dass das Hüllfluid (11) an das Probenfluid (10) in einem Angrenzungsbereich des Kanals direkt angrenzt und zumindest in dem Angrenzungsbereich in die gleiche Richtung wie das Probenfluid fließt,
wobei bei den im Kanal (12) auftretenden Scherraten des Hüllfluids (11) und des Probenfluids (10) die Viskosität des Hüllfluids (11) größer ist als die Viskosität des Probenfluids (10),
**dadurch gekennzeichnet, dass** die deformierbaren Körper durch die Kräfte, die sich in dem Probenfluid in dem Kanal (12) durch den Angrenzungsbereich einstellen, verformt werden, wobei die dynamische Viskosität des Hüllfluids (11) im Angrenzungsbereich im Bereich von 50 mPa s bis 250 mPa s liegt und wobei die dynamische Viskosität des Probenfluids (10) im Angrenzungsbereich im Bereich von 5 mPa s bis 50 mPa s liegt.

2. Verfahren nach Anspruch 1, wobei die durchschnittliche Flussgeschwindigkeit des Probenfluids (10) im Bereich von 0,1 cm/s bis 10 m/s liegt und/oder wobei die durchschnittliche Flussgeschwindigkeit des Hüllfluids (11) im Bereich von 0,1 cm/s bis 10 m/s liegt.

3. Verfahren nach einem der vorhergehenden Ansprüche, wobei das Probenfluid eine scherverdünnende Flüssigkeit ist, welche die deformierbaren Körper (14) enthält.

4. Verfahren nach einem der vorhergehenden Ansprüche, wobei das Probenfluid (10) und das Hüllfluid (11) aus Flüssigkeiten bestehen, die sich zumindest in der Zeitskala, in der die deformierbaren Körper (14) den Angrenzungsbereich durchqueren, nicht oder nur unwesentlich vermischen.

5. Verfahren nach einem der vorhergehenden Ansprüche, wobei das Hüllfluid (11) eine newtonsche oder scherverdickende Flüssigkeit ist.

6. Verfahren zum Bestimmen der mechanischen Eigenschaften von deformierbaren Körpern, bevorzugt Zellen, wobei deformierbare Körper mittels des Verfahrens nach einem der vorhergehenden Ansprüche verformt werden und wobei die Verformung der deformierbaren Körper bevorzugt mittels eines optischen Verfahrens gemessen wird.

7. Verfahren zum Untersuchen der zellbiologischen Eigenschaften von Zellen, umfassend das Verformen von Zellen mit dem Verfahren nach einem der Ansprüche 1 bis 6 und dem Untersuchen der biochemischen Eigenschaften von Zellkomponenten, insbesondere des Zytoskeletts oder der Organellen, aufgrund von Veränderungen in diesen Zellkomponenten durch die Verformung, wobei die Veränderungen bevorzugt durch ein fluoreszenzbasiertes Verfahren, insbesondere Durchflusszytometrie, gemessen werden.

8. Verfahren zum Sortieren von verformbaren Körpern, insbesondere Zellen, aufweisend:
- Durchführen des Verfahrens nach einem der Ansprüche 6 und/oder 7 und
- Sortieren von Zellen aufgrund der bestimmten mechanischen und/oder zellbiologischen Eigenschaften, bevorzugt mit einem Durchflusszytometer mit Sortierfunktion.

9. Verfahren nach einem der vorhergehenden Ansprüche, wobei vor Beginn des Messens der Verformung der deformierbaren Körper die Gesamtflussrate von Probenfluid und Hüllfluid von einem Anfangswert auf einen höheren Zielwert vergrößert wird.

## Claims

1. Method for deforming deformable bodies (14), preferably droplets or cells, having the following steps:
- feeding a sample fluid (10) into a channel (12), such that a laminar flow of the sample fluid results, wherein the sample fluid (10) transports deformable bodies,
- feeding a sheath fluid (11) into the channel (12), such that a laminar flow of the sheath fluid (11) results, and such that the sheath fluid (11) directly borders the sample fluid (10) in a border region of the channel and, at least in the border region, flows in the same direction as the sample fluid,
wherein, at the shear rates of the sheath fluid (11) and sample fluid (10) occurring in the channel (12), the viscosity of the sheath fluid (11) is greater than the viscosity of the sample fluid (10),
**characterised in that** the deformable bodies are deformed by the forces setting in the sample fluid in the channel (12) due to the border region, wherein the dynamic viscosity of the sheath fluid (11) in the border region is within a range of 50 mPa.s to 250 mPa.s and wherein the dynamic viscosity of the sample fluid (10) in the border region is within a range of 5 mPa.s to 50 mPa.s.

2. Method according to claim 1, wherein the average flow rate of the sample fluid (10) is within a range of 0.1 cm/s to 10 m/s and/or wherein the average flow rate of the sheath fluid (11) is within a range of 0.1 cm/s to 10 m/s.

3. Method according to any one of the preceding claims, wherein the sample fluid is a shear-thinning liquid which contains the deformable bodies (14).

4. Method according to any one of the preceding claims, wherein the sample fluid (10) and the sheath fluid (11) consist of liquids which, at least within the timescale during which the deformable bodies (14) traverse the border region, do not, or only to a minor extent, mix.

5. Method according to any one of the preceding claims, wherein the sheath fluid (11) is a Newtonian or shear-thickening liquid.

6. Method for determining the mechanical properties of deformable bodies, preferably cells, wherein deformable bodies are deformed using the method according to any one of the preceding claims and wherein the deformation of the deformable bodies is preferably measured using an optical method.

7. Method for examining the cell biological properties of cells, comprising the deformation of cells with the method according to any one of the claims 1 to 6 and the examination of the biochemical properties of cell components, in particular the cytoskeleton or the organelles, based on changes in these cell components due to the deformation, wherein the changes are preferably measured using a fluorescence-based method, in particular flow cytometry.

8. Method for sorting deformable bodies, in particular cells, consisting of:
- performing the method according to any one of the claims 6 and/or 7, and
- sorting cells, based on the determined mechanical and/or cell biological properties, preferably with a flow cytometer with sorting function.

9. Method according to any one of the preceding claims, wherein, prior to the start of measuring the deformation of the deformable bodies, the total flow rate of the sample fluid and sheath fluid is increased from an initial value to a higher target value.

## Revendications

1. Procédé de déformation de corps déformables (14), de préférence de gouttelettes ou de cellules, avec les étapes suivantes :
la fourniture d'un fluide échantillon (10) dans un canal (12) de sorte qu'un flux laminaire du fluide échantillon apparaisse, dans lequel le fluide échantillon (10) transporte des corps déformables,
- la fourniture d'un fluide d'enveloppe (11) dans le canal (12) de sorte qu'un flux laminaire du fluide d'enveloppe (11) apparaisse et de sorte que le fluide d'enveloppe (11) soit directement adjacent au fluide échantillon (10) dans une zone adjacente du canal et s'écoule au moins dans la zone adjacente dans la même direction que le fluide échantillon,
dans lequel pour les taux de cisaillement survenant dans le canal (12) du fluide d'enveloppe (11) et du fluide échantillon (10), la viscosité du fluide d'enveloppe (11) est supérieure à la viscosité du fluide échantillon (10),
**caractérisé en ce que** les corps déformables sont déformés par les forces qui se produisent dans le fluide échantillon dans le canal (12) par la zone adjacente, dans lequel la viscosité dynamique du fluide d'enveloppe (11) se trouve dans la zone adjacente dans la plage de 50 mPa s à 250 mPa s et dans lequel la viscosité dynamique du fluide échantillon (10) se trouve dans la zone adjacente dans la plage de 5 mPa s à 50 mPa s.

2. Procédé selon la revendication 1, dans lequel la vitesse de flux moyenne du fluide échantillon (10) se trouve dans la plage de 0,1 cm/s à 10 m/s et/ou dans lequel la vitesse de flux moyenne du fluide enveloppe (11) se trouve dans la plage de 0,1 cm/s à 10 m/s.

3. Procédé selon l'une des revendications précédentes, dans lequel le fluide échantillon est un liquide se diluant au cisaillement qui contient les corps déformables (14).

4. Procédé selon l'une des revendications précédentes, dans lequel le fluide échantillon (10) et le fluide d'enveloppe (11) se composent de liquides qui ne se mélangent pas ou que légèrement au moins dans l'échelle de temps, dans laquelle les corps déformables (14) traversent la zone adjacente.

5. Procédé selon l'une des revendications précédentes, dans lequel le fluide d'enveloppe (11) est un liquide newtonien ou s'épaississant au cisaillement.

6. Procédé de détermination des propriétés mécaniques de corps déformables, de préférence de cellules, dans lequel des corps déformables sont déformés au moyen du procédé selon l'une des revendications précédentes et dans lequel la déformation des corps déformables est mesurée de préférence au moyen d'un procédé optique.

7. Procédé d'examen des propriétés de biologie cellulaire de cellules, comprenant la déformation de cellules avec le procédé selon l'une des revendications 1 à 6 et l'examen des propriétés biochimiques de composants cellulaires, en particulier du cytosquelette ou des organelles, en raison de modifications de ces composants cellulaires par la déformation, dans lequel les modifications sont de préférence mesurées par un procédé basé sur la fluorescence, en particulier la cytométrie en flux.

8. Procédé de tri de corps déformables, en particulier de cellules, présentant :
- la réalisation du procédé selon l'une des revendications 6 et/ou 7 et
- le tri de cellules en raison de propriétés mécaniques et/ou de biologie cellulaire déterminées, de préférence avec un cytomètre en flux avec une fonction de tri.

9. Procédé selon l'une des revendications précédentes, dans lequel avant le début de la mesure de la déformation des corps déformables, le débit global du fluide échantillon et du fluide d'enveloppe est augmenté d'une valeur de départ à une valeur cible supérieure.
